(19) Europäisches Patentamt
European Patent Office
Office européen des brevets

(11) Veröffentlichungsnummer: **0 199 002**
**B1**

(12) **EUROPÄISCHE PATENTSCHRIFT**

(45) Veröffentlichungstag der Patentschrift: **16.05.90**

(51) Int. Cl.5: **C 07 C 69/75,** C 07 C 67/44

(21) Anmeldenummer: **86101560.0**

(22) Anmeldetag: **06.02.86**

# (54) Verfahren zur Herstellung von Delta3-Tetrahydrobenzoesäure-Delta3-tetrahydrobezylester.

(30) Priorität: **25.04.85 DE 3514938**

(43) Veröffentlichungstag der Anmeldung:
**29.10.86 Patentblatt 86/44**

(45) Bekanntmachung des Hinweises auf die Patenterteilung:
**16.05.90 Patentblatt 90/20**

(84) Benannte Vertragsstaaten:
**AT CH DE FR GB IT LI NL SE**

(56) Entgegenhaltungen:
**FR-A-1 156 046**

(73) Patentinhaber: **Degussa Aktiengesellschaft**
**Weissfrauenstrasse 9.**
**D-6000 Frankfurt am Main 1 (DE)**

(72) Erfinder: **Prescher, Günter, Dr. Dipl.-Chem.**
**Liesingstrasse 2**
**D-6450 Hanau 9 (DE)**
Erfinder: **Grund, Andreas, Dr. Dipl.-Chem.**
**Rilkeweg 15**
**D-6100 Darmstadt (DE)**
Erfinder: **Pesch, Heinrich, Ing.-grad.**
**Tulpenstrasse 23**
**D-6450 Hanau 8 (DE)**
Erfinder: **Böhme, Georg**
**Nordring 49**
**D-6458 Rodenbach (DE)**

EP 0 199 002 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Verfahren zur Herstellung von Δ3-Tetrahydrobenzoesäure-Δ3-tetrahydrobenzylester.

Die vorliegende Erfindung betrifft ein Verfahren zur kontinuierlichen Herstellung von Δ3-Tetrahydrobenzoesäure-Δ3-tetrahydrobenzylester.

Es ist bekannt, daß sich aliphatische als auch aromatische Aldehyde schonend in Gegenwart von Aluminiumoder Magnesiumalkoholaten in Ester überführen lassen, die formal aus einem dem Aldehyd entsprechenden Alkohol und der Carbonsäure des gleichen Kohlenstoffgerüsts gebildet werden. (V. Tischtschenko et al., Chem, Zentralblatt, 1309, 1554, 1556; (1906)).

In der US—PS 2 698 339 wird die Herstellung von Δ3-Tetrahydrobenzoesäure-Δ3-tetrahydrobenzylester aus 3-Cyclohexen-1-carboxaldehyd in Gegenwart von Aluminiumalkoholaten beschrieben. Gemäß dieser Vorschrift ist es empfehlenswert, mit frisch destilliertem Aldehyd zu arbeiten, da ansonsten erhöhte Mengen an Katalysator bei gleichzeitig schlechteren Ausbeuten notwendig sind.

Ein ähnliches absatzweises Verfahren wird in der US—PS 3 048 628 beschrieben. Auch hier erfolgt die Umsetzung von 3-Cyclohexen-1-carboxaldehyd mit Aluminiumtriisopropylat, welches zuvor in stark verdünnter Lösung im Reaktor vorgelegt und nach der Umsetzung mit Essigsäure hydrolysiert wird, wobei der entstehende voluminöse Niederschlag von Aluminiumhydroxid vor der Aufarbeitung entfernt werden muß.

Die US-PS 3 709 923 (vgl. P.R. Stapp, J. Org. Chem., *38,* 1433 (1973)) betrifft die Herstellung von Δ3-Tetrahydrobenzoesäure-Δ3-tetrahydrobenzylester mit Bor-haltigen Katalysatorsystemen. Da jedoch die Ausbeuten niedriger als 79% sind und zudem die Anwesenheit eines Lösungsmittels erforderlich ist, stellt dieses Verfahren keine wirtschaftlich interessante Alternative dar.

Aufgabe der Erfindung ist ein kontinuierliches Verfahren zur Herstellung von Δ3-Tetrahydrobenzoesäure-Δ3-tetrahydrobenzylester, bei dem auch nicht frisch destillierter 3-Cyclohexen-1-carboxaldehyd mit gutem Erfolg einzusetzen ist.

Gegenstand der Erfindung ist ein Verfahren zur Herstellung von Δ3-Tetrahydrobenzoesäure-Δ3-tetrahydrobenzylester (THB-Ester) durch Umsetzung von 3-Cyclohexen-1-carboxaldehyd (THB) mit Aluminiumalkoholaten, das dadurch gekennzeichnet ist, daß man mindestens 90 Gew.- %igen 3-Cyclohexen-1-carboxaldehyd zusammen mit 0,1 bis 5, bevorzugt 0,5 bis <1 Gew.%, bezogen auf den Aldehyd, eines flüssigen Aluminiumalkoholats oder -Mischalkoholats bei 20 bis 100°C, bevorzugt bei 30 bis 50°C, kontinuierlich in eine Kaskade, bestehend aus 1 bis 3 Reaktoren mit quasi vollständiger Rückvermischung, einspeist, anschließend bei 20 bis 100°C einem Strömungsrohr zuführt und das Reaktionsgemisch destillativ aufarbeitet.

Die Verweilzeit stellt man so ein, daß der Umsatz an 3-Cyclohexen-1-carboxaldehyd nach der Kaskade über 90%, vorzugsweise über 95% und hinter dem Nachreaktor über 96%, vorzugsweise über 98%, liegt.

Die reaktion läuft nach folgendem Schema ab

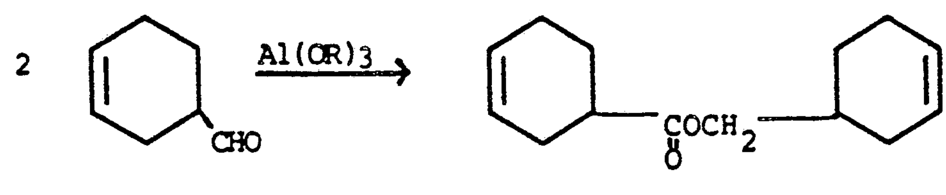


Bevorzugt eingesetzt wird ein flüssiges Mischalkoholat der Formel $Al(OR)_3$, dessen Alkoholkomponenten jeweils zur Hälfte aus Isoproylat und sek.-Butylatgruppen besteht.

In Gegensatz zu bekannten Verfahren reicht es aus, wenn der 3-Cyclohexen-1-carboxaldehyd mindestens 90 Gew.%ig und nicht frisch destilliert ist. Es ist daher besonders vorteilhaft, das aus der Umsetzung von Butadien und Acrolein stammende Reaktionsgemisch, das überwiegend aus 3-Cyclohexen-1-carboxaldehyd besteht, einzusetzen.

Nach dem Durchlaufen der Reaktorkaskade beläuft sich der Umsatz im Reaktionsgemisch an THB auf mehr als 95% und steigt aufgrund der Nachreaktion im Strömungsrohr ohne Rückvermischung auf über 98% an.

Da der Katalysator hydrolyseempfindlich ist, muß auf Wasserausschluß im eingesetzten THB sowie in den Reaktorgasräumen geachtet werden. Die Reaktion wird unter Normaldruck durchgeführt.

Die Aufarbeitung erfolgt destillativ, beispielsweise in einer einzigen Destillationskolonne, die bei vermindertem Druck, vorzugsweise 1—20 mbar betrieben wird. Leichtsiedende Nebenprodukte werden über Kopf abgenommen; des Hauptprodukt THB-Ester wird dampfförmig im Seitenstrom zwischen Einspeisung und Sumpf abgezogen. Der hochsiedene Katalysator wird neben hochsiedenden Nebenprodukten im Sumpf auf ca. 65—70% aufkonzentriert; der Sumpfabzug wird dann über einen Dünnschichtverdampfer noch einmal soweit zur Rückgewinnung von THB-Ester aufkonzentriert, daß der Sumpfabzug des Dünnschichtverdampfers noch fließfähig bleibt.

Die bilanzierte Gesamtausbeute an THB--Ester, bezogen auf THB, beträgt 95—96%.

Zur Erläuterung sei angemerkt, daß der Katalysator nach der Reaktion mit

R = $-CH_2-$ 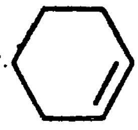

vorliegt.

Beispiel 1

In einer zweistufigen Rührkesselkaskade, die aus zwei Glasgefäßen von 560 ml und 690 ml Inhalt sowie einem nachgeschalteten Rohrreaktor vom Volumen 260 ml bestand, wurden über zwei getrennte Zuleitungen pro Stunde 484,0 g 3-Cyclohexen-1-carboxaldehyd (98,4%) und 6,16 g Aluminiummischalkoholat, was einem Katalysatoranteil von 1,27 Gew.-% entspricht, in den ersten Rührkessel eingespeist. Bei einer Reaktionstemperatur von 41°C im ersten Reaktor, 41°C im zweiten Reaktor und 43°C im Nachreaktor resultierten Umsätze an 3-Cyclohexen-1-carboxaldehyd von 95,8% im ersten Reaktor, 98,8% im zweiten Reaktor und 99,7% im Nachreaktor Die Gesamtverweilzeit betrug ca. 3,1 h.

Das aus der Kaskade kommende Reaktionsgemisch wurde in eine Destillationskolonne (20 Glockenböden, DN 50) zwischen dem 10./11. Boden eingespeist. Bei einer Sumpftemperatur von 200—220°C und einem Druck von 7 mbar gingen am Kopf geringe Mengen an Leichtsiedern und nicht umgesetztem 3-Cyclohexen-1-carboxaldehyd über. Das Produkt Δ3-Tetrahydrobenzoesäure-Δ3-tetrahydrobenzylester wurde dampfförmig im Seitenstrom oberhalb des Sumpfs entnommen; stündlich fielen 453,2 g von 99,1% Reinheit (GC) an. Aus dem Sumpf der Destillation konnten über einen nachgeschalteten Dünnschichtverdampfer (5 mbar, 200°C) weitere 16,6 g (96%) Produkt pro Stunde gewonnen werden, so daß die isolierte Gesamtausbeute 96,1% beträgt.

Beispiel 2

In einer zweistufigen Rührkesselkaskade, die aus zwei Glasgefäßen von 560 ml und 690 ml Inhalt sowie einem nachgeschalteten Rohrreaktor vom Volumen 260 ml bestand, wurdenüber zwei getrennte Zuleitungen pro Stunde 564,9 g 3-Cyclohexen-1-carboxaldehyd (94,9%) und 9,85 g Aluminiummischalkoholat, was einem Katalysatoranteil von 1,74 Gew.-% entspricht, in den ersten Rührkessenl eingespeist. Bei einer Reaktionstemperatur von 64°C im ersten Reaktor, 60°C im zweiten Reaktor und 61°C im Nachreaktor resultierten Umsätze an 3-Cyclohexen-1-carboxaldehyd von 97,1% im ersten Reaktor, 98,9% im zweiten Reaktor und 99,8% im Nachreaktor. Die Gesamtverweilzeit betrug ca. 2,7 h.

Das aus der Kaskade kommende reaktionsgemisch wurde in eine Destillationskolonne (20 Glockenböden, DN 50) zwischen dem 10./11. Boden eingespeist. Bei einer Sumpftemperatur von 200—220°C und einem Druck von 7 mbar gingen am Kopf geringe Mengen an Leichtsiedern und nicht umgesetztem 3-Cyclohexen-1-carboxaldehyd über. Das Produkt Δ3-Tetrahydrobenzoesäure-Δ3-tetrahydrobenzylester wurde dampfförmig im Seitenstrom oberhalb des Sumpfs entnommen; stündlich fielen 487,8 g von 99,0% Reinheit (GC) an. Aus dem Sumpf der Destillation konnten über einen nachgeschalteten Dünnschichtverdampfer (5 mbar, 200°C weitere 20,3 g (95%) Produkt pro Stunde gewonnen werden, so daß die isolierte Gesamtausbeute 93,7% beträgt.

Beispiel 3

In einer zweistufigen Rührkesselkaskade, die aus zwei Glasgefäßen von 560 ml und 690 ml Inhalt sowie einem nachgeschalteten Rohrreaktor vom Volumen 260 ml bestand, wurdenüber zwei getrennte Zuleitungen pro Stunde 584,8 g 3-Cyclohexen-1-carboxaldehyd (93,6%) und 4,71 g Aluminiummischalkoholat, was einem Katalysatoranteil von 0,81 Gew.-% entspricht, in den ersten Rührkessel eingespeist. Bei einer Reaktionstemperatur von 41°C im ersten Reaktor, 40°C im zweiten Reaktor und 42°C im Nachreaktor resultierten Umsätze an 3-Cyclohexen-1-carboxaldehyd von 96,2% im ersten Reaktor, 97,9% im zweiten Reaktor und 99,5% im Nachreaktor. Die Gesamtverweilzeit betrug ca. 2,5 h.

Das aus der Kaskade kommende reaktionsgemisch wurde in eine Destillationskolonne (20 Glockenböden, DN 50) zwischen dem 10./11. Boden eingespeist. Bei einer Sumpftemperatur von 200—220°C und einem Druck von 7 mbar gingen am Kopf geringe Mengen an Leichtsiedern und nicht umgesetztem 3-Cyclohexen-1-carboxaldehyd über. Das Produkt Δ3-Tetrahydrobenzoesäure-Δ3-tetrahydrobenzylester wurde dampfförmig im Seitenstrom oberhalb des Sumpfs entnommen; stündlich fielen 499,7 g von 98,8% Reinheit (GC) an. Aus dem Sumpf der Destillation konnten über einen nachgeschalteten Dünnschichtverdampfer (5 mbar, 200°C) weitere 21,1 g (96%) Produkt pro Stunde gewonnen werden, so daß die isolierte Gesamtausbeute 93,9% beträgt.

**Patentansprüche**

1. Verfahren zur Herstellung von Δ3-Tetrahydrobenzoesäure-Δ3-tetrahydrobenzylester durch Umsetzung von 3-Cyclohexen-1-carboxaldehyd mit Aluminiumalkoholaten, dadurch gekennzeichnet, daß man mindestens 90 Gew.-%igen nicht frisch destillierten 3-Cyclohexen-1-carboxaldehyd mit 0,1 bis 5, bevorzugt 0,5 bis <1 Gew.%, bezogen auf den Aldehyd, eines bei Raumtemperatur flüssigen Aluminiumalkoholats oder -Mischalkoholats bei 20 bis 100°C, bevorzugt bei 30 bis 50°C, kontinuierlich in eine Kaskade, bestehend aus 1 bis 3 Reaktoren mit quasi vollständiger Rückvermischung, einspeist, anschließend bei 20 bis 100°C einem Strömungsrohr zuführt, wobei man die Verweilzeit so einstellt, daß der Umsatz an 3-Cyclohexen-1-carboxaldehyd nach der Kaskade über 90%, vorzugsweise über 95% und hinter dem Nachreaktor über 96%, vorzugsweise über 98% liegt, und das Reaktionsgemisch destillativ aufarbeitet.

2. Verfahren nach Anspruch 1, dadurch gekennzeichnet, daß man ein Aluminium-Mischalkoholat einsetzt, dessen Alkoholkomponenten aus Isopropylat- und sek.-Butylatgruppen bestehen.

**Revendications**

1. Procédé de préparation de Δ3-tétrahydrobenzoate acide Δ3-tétrahydrobenzyle ester par réaction de 3-cyclohexène-1-carboxaldéhyde avec des alcoolates d'aluminium, caractérisé en ce qu'on introduit en continu dans une cascade du 3-cyclohexéne-1-carboxaldéhyde à 90% au moins non distillé avec 0,1 à 5, de préférence 0,5 à <1% en poids, par rapport à l'aldéhyde d'un alcoolate ou alcoolate mixte d'aluminium liquide entre 20 et 100°C, de préférence entre 30 et 50°C, la cascade étant constituée de 1 à 3 réacteurs avec un remélange quasicomplet, et ensuite on alimente un tube d'écoulement entre 20 et 100°C, on règle le temps de séjour de sorte que le rendement par rapport au 3-cyclohexéne-1-carboxaldéhyde dépasse 90% après la cascade, de préférence 95% et après le réacteur final, 96%, de préférence 98%, et on traite le mélange réactionnel par distillation.

2. Procédé selon la revendication 1, caractérisé en ce qu'on utilise un alcoolate mixte d'aluminium dont les composants alcools sont constitués de groupes isopropylate et sec.-butylate.

**Claims**

1. A process for the preparation of Δ3-tetrahydrobenzoic acid-Δ3-tetrahydrobenzyl ester by reaction of 3-cyclo-hexene-1-carboxaldehyde with aluminium alcoholates, characterized in that at least 90% by weight of non-freshly distilled cyclohexene-1-carboxaldehyde is continuously introduced with 0.1 to 5% by weight and preferably with 0.5 to <1% by weight, based on the aldehyde, of an aluminium alcoholate or mixed alcoholate liquid at room temperature into a cascade consisting of 1 to 3 reactors with substantially complete remixing at a temperature of 20 to 100°C and preferably at a temperature of 30 to 50°C; the resulting mixture is subsequently delivered to a flow tube, the residence time being adjusted so that the conversion of 3-cyclohexene-1-carboxaldehyde is more than 90% and preferably more than 95% after the cascade and more than 96% and preferably more than 98% behind the after-reactor, and the reaction mixture is worked up by distillation.

2. A process as claimed in claim 1, characterized in that an aluminium mixed alcoholate of which the alcohol components consist of isopropylate and sec.-butylate groups is used.